# EUROPEAN PATENT SPECIFICATION

(11) **EP 2 534 948 B1**
(45) Date of publication and mention of the grant of the patent: **22.06.2016**
(21) Application number: 12169194.3
(22) Date of filing: 24.05.2012
(51) Int. Cl.: A01N 25/04, A01N 43/80

(54) **Method of preparation of aqueuous dispersions of 1,2-Benzisothiazoline-3-one**
Verfahren zur Herstellung von wässrigen Dispersionen von 1,2-Benzoisothiazolin-3-on
Procédé de préparation de dispersions aqueuses de 1,2-Benzisothiazoline-3-one

(30) Priority: 17.06.2011 US 201161498218 P; 26.01.2012 US 201261590978 P
(43) Date of publication of application: 19.12.2012
(73) Proprietor: Rohm and Haas Company, Philadelphia, PA 19106-2399 (US); Dow Global Technologies LLC, Midland, MI 48674 (US)
(72) Inventor: Lenoir, Pierre, Marie, Alain, 8805 Richterswil (CH); Felder, Patrick, Thomas, 9472 Grabs (CH); Mettler, Anton, Oskar, 9740 Buchs (CH)
(74) Representative: Hoggins, Mark Andrew

(56) References cited:
- EP-A1- 1 389 424
- WO-A1-03/032730
- WO-A1-2011/085067
- WO-A1-2011/150224
- WO-A2-95/00019
- ANONYMOUS: 'Safety data sheet POTASSIUM METABISULFITE', [Online] 19 March 2003, XP055204168 Retrieved from the Internet: <URL:http://bsgwine.com/PDF/Potassium_Meta_ MSDS.pdf> [retrieved on 2015-07-22]
- ANONYMOUS: 'Sodium lignosulfonate | 8061-51-6', [Online] 01 January 2010, XP055204174 Retrieved from the Internet: <URL:http://www.chemicalbook.com/ChemicalPr oductProperty_EN_CB9282256.htm> [retrieved on 2015-07-22]

## Description

The present invention pertains to Aqueous Dispersions.

The following is the background of the invention.

1,2-Benzisothiazolin-3-one (BIT) is an effective biocide that is employed, for example, for killing microorganisms (including, for example, bacteria, fungi, molds, yeasts, etc.) in useful aqueous compositions such as, for example, latices. BIT has low solubility in water when the pH is 7.5 or lower, and so it is common to provide BIT in the form (called an "aqueous BIT dispersion") of BIT particles dispersed in water. It is often desired to provide BIT in a form (called a "BIT concentrate") that has higher concentration of BIT than is needed for control of microorganisms in useful aqueous compositions. An appropriate amount of a BIT concentrate could be added to a useful aqueous composition to achieve the concentration of BIT that is needed for control of microorganisms in the useful aqueous composition. To be useful, a BIT dispersion concentrate (i.e., an aqueous BIT dispersion that is also a BIT concentrate) should have a useful viscosity, and it should be stable. A stable aqueous dispersion of BIT containing a metal salt of an organic acid such as potassium formate is disclosed in WO-A-03/032730.

In BIT dispersions, including BIT dispersion concentrates, it is usually desirable for the solid particles to have small size and for the dispersion to be stable. Typically, in the past, to make the particles sufficiently small, particles of BIT were subjected to a mechanical process (such as, for example, milling) to reduce the size of the particles. For example, WO 95/00019 discloses stable aqueous dispersions of BIT which contain Xanthan gum which are subjected to milling to provide dispersed matter having a particle size of less than 20 µm.

US Provisional Patent Application 61/371,811 describes a process for making an aqueous dispersion of particles of 1,2-Benzisothiazolin-3-one (BIT). The process described by US Provisional Patent Application 61/371,811 involves obtaining particles of BIT as they are normally manufactured, which are undesirably large, then milling those particles to reduce the size.

It is desired to provide a method of making a stable aqueous dispersion of BIT; it is desired that the method does not require milling and that the method produces a dispersion having desirably small particle size.

The present invention is as set out in the accompanying claims.

The present invention is a method of making an aqueous dispersion of BIT particles having a D99 particle size distribution, measured using light scattering, of 75 microns or smaller, wherein said dispersion comprises (A) one or more thickener and (B) solid particles of 1,2-benzisothiazolin-3-one, wherein said method is as set out in the accompanying claims.

The following is a detailed description of the invention.

As used herein, the following terms have the designated definitions, unless the context clearly indicates otherwise.

As used herein, a continuous liquid medium is an "aqueous medium" if the continuous liquid medium is 20% or more water, by weight based on the weight of the continuous liquid medium.

As used herein, a dispersion is a composition in which solid particles are distributed throughout a continuous liquid medium. An aqueous dispersion is a dispersion in which the continuous liquid medium is an aqueous medium. As used herein, a solution is different from a dispersion. In a solution, individual molecules of a compound (the solute) are distributed throughout a continuous liquid medium (the solvent). A solution is aqueous if the solvent is an aqueous medium.

As used herein, a solvent is a compound that is liquid at 25°C and has a boiling point at one atmosphere pressure of 30°C or higher. As used herein, a compound is soluble in a solvent if the amount of that compound that can be dissolved in that solvent at 25°C is one gram or more of that compound per 100 grams of that solvent.

When it is stated herein that a mixture of two or more components is formed (or, synonymously, that those two or more components are mixed together), it is meant that those components are brought into contact with each other and are blended together. Unless otherwise stated, the components may be introduced to each other in any order. For example, in a mixture of two components, the first component may be added to the second component; or the second component may be added to the first component; or the two components may be added simultaneously to a container; or some other method may be used. For another example, if a mixture of three or more components is made, a first mixture of any two or more components may be made and then a subsequent mixture may be made of that first mixture with one or more of the remaining components. Any one component may be added in two or more separate operations; for example, in a first step a first component may be mixed with some of a second component, and in a second step, the result of the first step may be mixed with a third component, and then the result of the second step may be mixed with a further amount of the second component.

As used herein, when two or more components are said to be "mixed" or to be "mixed together," it is meant that those components are brought into contact with each other for the first time.

A thickener is a compound or mixture of compounds that, when added to water, to an aqueous solution, or to an aqueous dispersion, causes an increase in viscosity. It is useful to characterize thickeners as either pH-sensitive or not pH-sensitive, according to the following test. 1 part by weight of thickener active ingredient is mixed with 100 parts by weight of water at pH of 7, and the steady-shear viscosity of the mixture is measured at 1 sec⁻¹ at pH of 7 (ETA7). Base is added to the mixture to bring pH to 10, and the steady-shear viscosity of the mixture is measured at 1 sec⁻¹ (ETA10). If the quotient of ETA10 divided by ETA7 is 5 or more, then the thickener is pH-sensitive. Otherwise, the thickener is not pH-sensitive.

The term "microbicide" or "biocide" refers to a compound capable of killing, inhibiting the growth of, or controlling the growth of microorganisms. Biocides include, for example, bactericides, fungicides and algicides. The term "microorganism" includes, for example, bacteria, fungi (such as yeast and mold), and algae.

A polysaccharide is a polymer made of more than 10 repeat units, where the repeat units are monosaccharides.

When it is stated herein that a certain ratio is "X:1 or higher," it is meant that the ratio is Y:1, where Y is greater than or equal to X. Similarly, when it is stated herein that a certain ratio is "Q:1 or lower," it is meant that the ratio is T:1, where T is less than or equal to Q.

As used herein, agitation is a mechanical action performed on a container that serves to distribute throughout the contents of the container each of the materials present within the container. Agitation includes, for example, processes that stir the contents of the container and processes that move the container. Stirring involves rotating one or more objects within the contents of the container; rotating objects include, for example, rods, impellers, screws, rotors, helices, etc. Processes that move the container include, for example, shaking, rolling, etc.

As used herein, "room temperature" is 25°C. As used herein, "pbw" is parts by weight.

The method of the present invention involves providing an aqueous mixture (herein called "aqueous mixture (I)") that contains water, a dissolved salt of BIT, and optionally one or more thickener and that has pH of 8.5 or higher. Preferably, aqueous mixture (I) is made by a process in which one or more base is added to the water or to a mixture of BIT, water, and optionally one or more thickener to establish the desired pH. Preferably, the base is soluble in water. Preferred bases are strong bases (i.e., bases with pK_{b} of 3 or lower). Preferred are inorganic bases. More preferred are water-soluble inorganic hydroxide salts; more preferred are calcium hydroxide, magnesium hydroxide, and water-soluble alkali metal hydroxide salts; more preferred are sodium hydroxide, potassium hydroxide, lithium hydroxide, and mixtures thereof.

In some embodiments (herein called "high-I" embodiments), aqueous mixture (I) contains one or more thickener. In some embodiments (herein called "low-I" embodiments), aqueous mixture (I) does not contain thickener.

Aqueous mixture (I) may be formed by any method.

Among low-I embodiments, for example, aqueous mixture (I) may be formed by making a solution of a base in water and then adding BIT. For another example among low-I embodiments, aqueous mixture (I) may be formed by making a mixture of BIT and water and then adding a base.

In high-I embodiments, a mixture (known herein as "mixture (III)") is formed. Mixture (III) contains water, BIT, and one or more thickener, and the pH of mixture (III) is 7.5 or lower. After formation of mixture (III), the pH of mixture (III) is raised to form aqueous mixture (I). Among high-I embodiments, for example, aqueous mixture (I) may be made by making a mixture of water and BIT, then adding one or more thickener, and then adding a base. For another example among high-I embodiments, aqueous mixture (I) may be made by making a mixture of water and one or more thickener, then adding BIT, and then adding a base.

Optionally, aqueous mixture (I) may contain one or more of the following: solvent other than water, base that has not reacted with BIT, BIT that has not reacted with base, other ingredients, and mixtures thereof.

The amount of base used in making aqueous mixture (I) is sufficient to bring the mixture to a pH value of 8.5 or higher; more preferably 11.0 or higher. Preferably, the pH of aqueous mixture (I) is 14 or lower.

BIT is normally provided by the manufacturer in the form of a collection of particles that are solid at 25°C. The particles in such a collection, including individual particles, aggregates, and agglomerates, range in size from 1 micrometer to 500 micrometer. Such a collection normally contains approximately 85% BIT and approximately 15% water, by weight. Such a collection appears to be a powder, though it is often (including herein) called a "paste."

In the practice of the present invention, BIT in any form may be used in the making of aqueous mixture (I). That is, when it is desired to make aqueous mixture (I), a person practicing the present invention may choose to begin with pure BIT, with BIT paste, with a solution of BIT in a solvent, with a dispersion of BIT, with some other form of BIT, or with a combination thereof. That is, the initial form of BIT does not matter, as long as the result qualifies as aqueous mixture (I) as defined herein. For example, BIT in the form of a paste supplied by the manufacturer may be added directly to water or to a solution of a base in water. Preferably, BIT paste is not subjected to any mechanical process (such as, for example, milling) for reducing particle size prior to use in making aqueous mixture (I). More preferably, BIT paste is not subjected to any process for reducing particle size prior to use in making aqueous mixture (I).

In the practice of the present invention, when BIT and base capable of reacting with BIT are both present in an aqueous medium, the base will react with BIT to form a salt of BIT. That salt of BIT will be soluble in the aqueous medium and will become a dissolved salt of BIT. Herein, when "dissolved BIT" or "BIT in solution" is mentioned, it is understood that the dissolved species is the salt of BIT.

Herein, when the "amount of BIT in solution" is mentioned, the amount that is meant is the amount of neutral BIT that is needed to make the solution. For example, if 5 grams of neutral BIT were used to make a solution that had total weight of 100 grams, herein the concentration of BIT in solution would be described as 5%.

The amount of BIT in the aqueous dispersion of the present invention is, by weight based on the weight of the aqueous dispersion, preferably 1% or more; more preferably 5% or more; more preferably 10% or more. The amount of BIT in the aqueous dispersion of the present invention is, by weight based on the weight of aqueous dispersion, is preferably 40% or less; more preferably 30% or less.

Preferably, the molar equivalent ratio of base to BIT used in making aqueous mixture (I) is 0.8:1 or higher; more preferably 0.9:1 or higher; more preferably 0.95:1 or higher; more preferably 0.99:1 or higher. Preferably, the molar equivalent ratio of base to BIT used in making aqueous mixture (I) is 5:1 or lower; more preferably 3:1 or lower; more preferably 1.5:1 or lower; more preferably 1.2:1 or lower; more preferably 1.1:1 or lower. As used herein, the molar equivalent ratio of base to BIT is the ratio of the moles of titratable base groups used in making aqueous mixture (I) to the moles of BIT used in making aqueous mixture (I). For example, if a monovalent base compound (such as, for example, an alkali hydroxide) is used, the number of moles of base groups is the number of moles of the monovalent base compound. For another example, if a divalent base compound (such as, for example, a compound with two hydroxide groups) is used, the number of moles of base groups is double the number moles of the divalent base compound.

The method of the present invention includes step (b), which involves forming a mixture (herein "aqueous mixture(II)") that contains aqueous mixture (I), optionally one or more thickener, and an acid. Thickener may be added during the formation of aqueous mixture (I) or during the formation of aqueous mixture (II). Aqueous mixture II may be made by combining the ingredients by any method that includes step (b1) which is performed prior to step (b2). Step (b1) is the step of making a mixture that contains a thickener and that contains aqueous mixture (I). If aqueous mixture (I) already contains one or more thickener from the performance of step (a), then further thickener may or may not be added during performance of step (b1). Such further thickener, if added, may be the same as or different from thickeners used in the performance of step (a), or a mixture thereof.

Step (b2) is the step of making a mixture that contains an acid and that contains aqueous mixture (I), where the amount of acid is sufficient to bring the mixture of acid and aqueous mixture (I) to pH of 1.5 to 7.5.

Among low-I embodiments, one or more thickener will be added to the composition during the formation of aqueous mixture (II). Among low-I embodiments, thickener is added to aqueous mixture (I) before acid is added. Among high-I embodiments, if additional thickener is added during formation of aqueous mixture (II), the additional thickener, the aqueous mixture (I), and the acid may be combined in any order.

Step (b2) is performed after step (b1), performing step (b2) involves combining an acid with the mixture resulting from step (b1).

In some embodiments, aqueous mixture (I) may be mixed with acid by adding a relatively small volume of aqueous mixture (I) that has relatively high concentration of dissolved salt of BIT into a relatively large volume of an aqueous solution of acid. In such embodiments, the concentration of BIT in aqueous mixture (I) is 5% or more; more preferably 10% or more; preferably 20% or more. In such embodiments, the volume of aqueous mixture (I) is smaller than the volume of the aqueous solution of acid.

It is useful to consider the form in which thickener exists at the moment when it is mixed with (i.e., brought into contact with) other ingredients of the composition of the present invention. That form may be any form, including, for example, pure thickener, a mixture of thickener with another composition, a solution of thickener in a solvent (triethylene glycol, for example), a dispersion of thickener particles in a liquid medium (aqueous medium, for example), or a mixture thereof. Similarly, it is useful to consider the form in which acid exists at the moment when it is mixed with (i.e., brought into contact with) other ingredients. That form may be any form, including, for example, pure acid, a mixture of acid with another composition, a concentrated solution of acid in a solvent (water, for example), a dilute solution of acid in a solvent, or a mixture thereof.

Any thickener is suitable. Suitable thickeners include, for example, polyelectrolytes (including, for example, homopolymers and copolymers that include polymerized units of acrylic acid and/or methacrylic acid), polysaccharides, cellulose ethers, derivatives of cellulose ethers, gums, and mixtures thereof. Preferred thickeners are not pH-sensitive. Preferred thickeners are soluble in water over a range of pH values that partially or fully lies within the range of pH from 1.5 to 10. Preferred thickeners are polysaccharides. Preferred are polysaccharides having 50 or more repeat units; more preferred are 75 or more repeat units. Preferred polysaccharides are water soluble. Preferred polysaccharides are cellulose ethers, derivatives of cellulose ethers, gums, and mixtures thereof.

Cellulose ethers and their derivatives are made by alkylation of cellulose and, optionally, additional chemical modification. Preferred cellulose ethers and their derivatives are water soluble. Among cellulose ethers and their derivatives, preferred are hydroxyethylcellulose, sodium carboxymethylcellulose, and hydroxypropylmethylcellulose; more preferred is hydroxyethylcellulose.

Gums are polysaccharides produced by biological systems. Gums may or may not receive treatment (e.g., addition of one or more chemical to the surface of gum particles). Preferred gums are water soluble. Preferred gums have weight-average molecular weight of 500,000 or more; more preferred is 1 million or more. The preferred gum is xanthan gum.

More preferred polysaccharides are hydroxyethylcellulose, xanthan gum, and mixtures thereof.

When one or more polysaccharide is present, the preferred amount of polysaccharide in the aqueous dispersion of the present invention, by weight based on the weight of the aqueous dispersion, is 0.3% or more; more preferably 0.5% or more; more preferably 0.6% or more. Independently, when one or more polysaccharide is present, the preferred amount of polysaccharide in the aqueous dispersion of the present invention, by weight based on the weight of the aqueous dispersion, is 2% or less; more preferably 1.5% or less; more preferably 1% or less. When a polysaccharide is present, the optimum amount depends on the specific polysaccharide that is used and on the amount of BIT.

In some embodiments, the aqueous dispersion of the present invention contains one or more secondary solvent selected from glycols and glycol ethers. When a secondary solvent is present, preferred are secondary solvents having the formula F1: 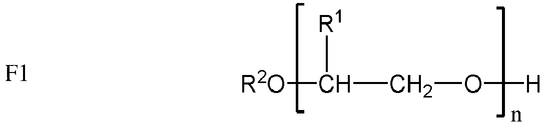 where n is 1 to 15; R¹ is hydrogen or alkyl; and R² is hydrogen, alkyl, aryl, substituted alkyl, or substituted aryl. When R² is hydrogen, the secondary solvent is a glycol. Preferred secondary solvents are glycols. Preferably, R¹ is hydrogen, methyl, or ethyl. More preferably, R¹ is hydrogen. Preferably, n is 2 to 10. When a secondary solvent is present, the preferred amount of secondary solvent, by weight based on the weight of the aqueous dispersion of the present invention is 0.1% or more; more preferably 0.5% or more; more preferably 1% or more; more preferably 2% or more. When a secondary solvent is present, the preferred amount of secondary solvent, by weight based on the weight of the aqueous dispersion of the present invention is 50% or less ; more preferably 25% or less; more preferably 10% or less; more preferably 6% or less. In some embodiments involving secondary solvent, the secondary solvent is mixed with aqueous mixture (I) either before thickener is mixed with aqueous mixture (I) or at the same time that thickener mixed with aqueous mixture (I). In some embodiments in which secondary solvent is used, a mixture (herein "2ST" mixture) of secondary solvent and thickener is made, and then that 2ST mixture is mixed with other ingredients. When a 2ST mixture is used, the amount of secondary solvent, by weight based on the weight of the aqueous dispersion of the present invention, is preferably 1% or more; more preferably 2% or more. When a 2ST mixture is used, the amount of secondary solvent, by weight based on the weight of the aqueous dispersion of the present invention, is preferably 8% or less; more preferably 6% or less. When a 2ST mixture is used, the 2ST mixture may or may not also contain some or all of the acid used in step (b) of the present invention.

The method of the present invention involves forming an aqueous dispersion by a process that comprises mixing acid with a mixture herein called the "target mixture." The target mixture contains aqueous mixture (I); the target mixture contains a thickener.

Any acid is suitable if it is capable of lowering the pH of an aqueous composition to which it is added. The pKₐ of the acid is preferably 7 or lower; more preferably 6 or lower; more preferably 5 or lower.

Suitable acids are either organic or inorganic. Suitable acids include organic acids, inorganic acids, phosphoric acids, and sulfonic acids. Preferred are inorganic acids, organic carboxylic acids, organic polycarboxylic acids, and organic sulfonic acids; more preferred are inorganic acids and alkyl carboxylic acids. Preferably, acid is in the form of a solution of the acid in water, and that aqueous solution of acid is added to target mixture.

It is useful to consider the speed with which acid is added. The speed of addition may be characterized by the acid speed unit ("asu") defined herein as moles of acid per minute per kg of BIT (i.e., kg of BIT that is in target mixture prior to addition of acid). Preferably, the speed of addition of acid is preferably 1.0 asu or higher; more preferably 2 asu or higher; more preferably 5 asu or higher; more preferably 10 asu or higher. It is acceptable to use a speed of addition of acid that is as high as possible, using any conventional means for adding acid to target mixture.

The amount of acid mixed with target mixture brings the pH to a value of 1.5 to 7.5.

Preferably, while acid is being mixed with target mixture, the mixture of acid and target mixture is agitated. That is, the mixture is preferably agitated as the mixing of acid and target mixture begins, and agitation preferably continues as all of the acid is mixed with all of target mixture. Preferably, agitation is performed by rotating an impeller within the mixture of acid and target mixture.

When agitation is performed by rotating an impeller with a liquid composition, it is useful to characterize the agitation by the tangential velocity of the tip of the impeller.

Preferably, mixing of acid with target mixture is performed while agitation is performed by rotating an impeller within the mixture at a tangential velocity of the tip of the impeller of 600 mm/sec or higher; more preferably 900 mm/sec or higher. Preferably, mixing of acid with target mixture is performed while agitation is performed by rotating an impeller within the mixture at a tangential velocity of the tip of the impeller of 6,500 mm/sec or lower; more preferably 5,000 mm/sec or lower.

In the practice of the present invention, when BIT, water, and optionally one or more thickener are brought together to form a mixture in which the pH is 8.5 or higher, the process is conducted in such a way that dissolved salt of BIT is formed. In some cases, mixing BIT, a base, water, and optionally one or more thickener in the appropriate amounts will result in a solution of dissolved salt of BIT, without the need for applying heat to the mixture. In some of such cases, the formation of the dissolved salt of BIT is an exothermic process, and the mixture reaches a temperature above the temperature at which the components were mixed, even if no heat is applied from an external source. Whether or not external heat is necessary for the formation of dissolved salt of BIT it is within the scope of the present invention to apply heat to the mixture of water, base, BIT, and optionally one or more thickener during the performance of step (a). Typically, at the moment that step (a) is completed, the result is aqueous mixture (I), in which BIT salt is dissolved, and aqueous mixture (I) may be at room temperature or may be at a temperature above room temperature.

Some thickeners have a characteristic labeled herein as "susceptible to high pH." Such thickeners do not easily dissolve in water at pH of 8.5 or higher. When such thickeners are added to water at pH of 8.5 or higher, lumps form, and extensive agitation is required to dissolve the lumps. A thickener that is susceptible to high pH may or may not be pH-sensitive. Xanthan gum is normally susceptible to high pH. Some commercial grades of xanthan gum are treated in an effort to render the xanthan gum not susceptible to high pH. Grades of xanthan gum that are not thus treated are susceptible to high pH.

When a thickener is used that is not susceptible to high pH, it is preferred to add that thickener to the composition during the formation of aqueous mixture (II). After the thickener is added, the increased viscosity of the composition will require increased energy to stir or otherwise agitate the composition, and so it is desired to add the thickener later in the process (i.e., during the formation of aqueous mixture (II)). When a thickener is used that is not susceptible to high pH, and when that thickener is added to the composition during the formation of aqueous mixture (II) the thickener is added to the composition while the pH is 8.5 or higher; that is, the thickener is added to the composition prior to adding acid.

When a thickener is used that is susceptible to high pH, it is preferred to practice the present invention in a high-I embodiment. When a thickener is used that is susceptible to high pH, it is more preferred to form a mixture of BIT, water, and such thickener at pH below 7.5, prior to the step of adding base during the formation of aqueous mixture (I).

Aqueous mixture (I) will possess an inherent precipitation temperature. That is, if the temperature of aqueous mixture (I) were to be lowered, a temperature would be reached (the inherent precipitation temperature) at which some of the salt of BIT would no longer be soluble, and some of the salt of BIT would precipitate. If the aqueous mixture (I) were to be lowered to the freezing point of aqueous mixture (I) without the occurrence of precipitation of any salt of BIT, then the freezing point of aqueous mixture (I) would be considered to be the inherent precipitation temperature. The inherent precipitation temperature is a property of the aqueous mixture (I) and depends, among other variables, on the type of base, the concentration of BIT and/or the concentration of dissolved salt of BIT.

In some embodiments, an aqueous mixture (I) is made that has an inherent precipitation temperature that is higher than room temperature. It is possible, using the methods of the present invention, to make an aqueous dispersion at a certain concentration of BIT that exists and is stable at room temperature, even though aqueous mixture (I) at that concentration of BIT has inherent precipitation temperature that is above room temperature. Such an aqueous dispersion would be made as follows. Aqueous mixture (I) is prepared by performing step (a), if necessary applying heat to the mixture. At the conclusion of step (a), the BIT is dissolved, and aqueous mixture (I) is at a temperature above the inherent precipitation temperature. Then, by applying heat to the composition as necessary, the composition is maintained at a temperature above the inherent precipitation temperature during the performance of step (b). At the conclusion of step (b), the BIT has precipitated and formed an aqueous dispersion, so the inherent precipitation temperature of aqueous mixture (I) is no longer relevant, because aqueous mixture (I) no longer exits. The aqueous dispersion may then be cooled to room temperature, and the aqueous dispersion will be stable at room temperature.

Embodiments are contemplated in which step (b1) and step (b2) are performed at 20°C to 60°C.

It is useful to contemplate a salt that has the composition of the salt that would be formed from the neutralization reaction between a base used in step (a) and an acid used in step (b). It is contemplated that one or more such salt will be formed by performing the process of the present invention. Such a salt formed by the process of the present invention is herein labeled a "process neutralization salt." It is contemplated that more than one process neutralization salt will be formed if more than one base is used in step (a); or if more than one acid is used in step (b); or if more than one base is used in step (a) and more than one acid is used in step (b). In some embodiments, one or more process neutralization salt will precipitate and will not be considered herein to be contained in the aqueous dispersion. In preferred embodiments, one or more process neutralization salt will remain dissolved or dispersed in the aqueous dispersion of the present invention, and these salts are considered herein to be contained in the aqueous dispersion. More preferably, all process neutralization salts will remain dissolved or dispersed in the aqueous dispersion of the present invention.

The total amount of all process neutralization salts contained in the aqueous dispersion of the present invention may be characterized by the ratio of the total number of equivalents of all process neutralization salts contained in the aqueous dispersion to the number of equivalents of BIT. Preferably, that ratio is 0.3:1 or higher; more preferably 0.5:1 or higher; more preferably 0.8:1 or higher; more preferably 0.9:1 or higher; more preferably 0.95:1 or higher; more preferably 0.99:1 or higher. Preferably, that ratio is 5:1 or lower; more preferably 3:1 or lower; more preferably 1.5:1 or lower; more preferably 1.2:1 or lower; more preferably 1.1:1 or lower.

The viscosity of the aqueous dispersion of the present invention may be characterized by Krebs units (KU), which is the result of viscosity measurements using a Stormer viscometer as defined in ASTM D562-10 (ASTM International, 100 Barr Harbor Drive, PO Box C700, West Conshohocken, PA 19428-2959, USA). Preferably, the viscosity of the aqueous dispersion of the present invention is 50 KU or higher; more preferably 60 KU or higher; more preferably 65 KU or higher. Preferably, the viscosity of the aqueous dispersion of the present invention is 100 KU or lower; more preferably 90 KU or lower; more preferably 80 KU or lower.

The particle size distribution of the BIT particles in the aqueous dispersion of the present invention is measured by laser light scattering. One suitable apparatus for such measurements is the Cilas particle size analyzer, Model Number 1064LD, Series Number Rev.1.02 (Cilas Particle Size, Madison, WI, USA). One way to assess the particle size distribution is D99, which is the smallest particle diameter that can be chosen so that 99% or more of the mass of the BIT particles, based on the mass of all the BIT particles in the aqueous dispersion, has particle diameter equal to or less than D99.

D99 is 75 micrometers or smaller; preferably 60 micrometers or smaller; more preferably 55 micrometers or smaller; more preferably 50 micrometers or smaller; more preferably 45 micrometers or smaller; more preferably 40 micrometers or smaller. Preferably D99 is 1 micrometer or larger; more preferably 5 micrometer or larger; more preferably 10 micrometer or larger.

The aqueous dispersion of the present invention has a continuous liquid medium that is 20% or more water, by weight based on the weight of the continuous liquid medium. Preferably, the amount of water in the continuous liquid medium is, by weight based on the weight of the continuous liquid medium, 50% or more; more preferably 75% or more; more preferably 85% or more; more preferably 95% or more. In some embodiments, the aqueous dispersion of the present invention contains one or more "additional solvent" (i.e., a solvent that is not water and is not any of the secondary solvents described herein above). The amount of all additional solvent, by weight based on the weight of the continuous liquid medium, is preferably 0% to 50%; more preferably 0% to 20%; more preferably 0% to 10%; more preferably 0% to 5%.

Some compositions of the present invention contain, in addition to BIT, one or more additional biocides. Additional biocides (i.e., biocides other than BIT) may be chosen, for example, from derivatives of 3-isothiazolone other than BIT, formaldehyde-releasing biocides, aldehydes, urea-based biocides, quaternary ammonium biocides, phenolic biocides, halogen-containing biocides, organometallic biocides, organosulfur biocides, heterocyclic biocides, biguanide biocides, other nitrogen-containing biocides, alcohol biocides, and mixtures thereof. When one or more additional biocide is present, preferred additional biocides are biocides that are able to be dissolved or dispersed in the aqueous medium of the present invention. When one or more additional biocide is present, preferred additional biocides are biocides that are stable (i.e., that retain biocidal activity when stored for 2 weeks or more at room temperature) in water over a range of pH (herein the "stability range"), where some or all of that stability range falls within the pH range of 1.5 to 7.5; it is contemplated that such biocides will be stable in the aqueous dispersion of the present invention. Some suitable additional biocides are water-soluble. Some suitable water-soluble additional biocides are, for example, water-soluble derivatives of 3-isothiazolone. Suitable water-soluble additional biocides include, for example, 5-chloro-2-methyl-4-isothiazolin-3-one (CMI), 2-methyl-4-isothiazolin-3-one (MI), bronopol, Dimethylol Dimethyl Hydantoin (DMDMH), ethylenedioxydimethanol (EDDM), glutaraldehyde, glyoxal, Tetrahydro-1,3,4,6-tetrakis(hydroxymethyl)imidazo(4,5-d)imidazole-2,5(1H,3H)-dione (CAS Registry number 5395-50-6, also called tetramethylolacetylene diurea or TMDU), and mixtures thereof..

When one or more water-soluble additional biocide is used, any amount of water-soluble biocide above zero is contemplated. When one or more water-soluble additional biocide is used, the preferred amount of water-soluble additional biocide is, by weight based on the weight of the aqueous dispersion, 0.3% or more; more preferred is 1% or more; more preferred is 2% or more; more preferred is 5% or more. Independently, when one or more water-soluble additional biocide is used, the preferred amount of water-soluble additional biocide is, by weight based on the weight of the aqueous dispersion, 25% or less; more preferred is 15% or less; more preferred is 12% or less; more preferred is 10% or less. When CMI or a mixture of CMI and MI is included, the preferred amount of CMI or of the mixture of CMI and MI is 0.3% to 2% by weight based on the weight of the aqueous dispersion.

In some of the embodiments in which one or more water-soluble additional biocide is used, one or more of the water-soluble additional biocides may be sensitive to the pH of the composition. That is, at pH above 5, such biocides become increasingly unstable as the pH is raised. In such embodiments, the pH is preferably 7.5 or lower; more preferably 6.0 or lower; more preferably 5.0 or lower.

In some embodiments, the composition contains, in addition to BIT, one or more biocide that is not soluble in water. Suitable biocides that are not BIT and that are not soluble in water include, for example, 2-n-octyl-4-isothiazolin-3-one (OIT), iodopropynyl butylcarbamate (IPBC), and ortho-phenylphenol (OPP). For example, one or more biocide other than BIT may be present in the form of dispersed liquid droplets or dispersed solid particles. Among such embodiments, the preferred amount of biocide that is not BIT and that is not soluble in water is 15% or less by weight based on the weight of the composition.

Whether any biocide is or is not present in addition to BIT, the preferred amount of total biocide (i.e., the sum of the amounts of all biocides) in the composition of the present invention is, by weight based on the weight of the composition of the present invention, 1% or more; more preferably 2% or more; more preferably 5% or more. Whether any biocide is or is not present in addition to BIT, the preferred amount of total biocide (i.e., the sum of the amounts of all biocides) in the composition of the present invention is, by weight based on the weight of the composition of the present invention, 50% or less; more preferably 35% or less; more preferably 25% or less.

If any additional biocide is present in the aqueous dispersion of the present invention, the additional biocide may be added at any point in the process of the present invention. For example, a water-soluble additional biocide may be added to water prior to the start of the process of the present invention or may be added to the appropriate solution or mixture before, during, or after any of step (a) or step (b). If a water-soluble additional biocide is used that is sensitive to pH (as defined herein above), it is preferred that such additional biocide is added after step (b).

In some embodiments, one or more adjuvants (i.e., ingredients other than those discussed herein above) is included in the aqueous dispersion of the present invention. Suitable adjuvants include, for example, surfactants, wetting agents, dispersants, chelants, fillers, defoamers, and mixtures thereof. Fillers are mineral powders such as, for example, titanium dioxide and clay.

The following are examples of the present invention.

In the following examples, operations are performed at approximately 23°C unless stated otherwise. BIT paste was used without any milling or other mechanical process to reduce particle size.

In some of the following Examples, "Protocol 1" was performed. Parts of Protocol 1 follow the steps of the method of the present invention, but other parts of Protocol 1 were performed in order to determine certain characteristics of the compositions being studied. Those characteristics provide information about which mixtures can or cannot be used in the method of the present invention.

"Protocol 1" means the following: A mixture (herein "mixture 1") was formed as follows: 151.18 g of BIT paste (concentration 83% to 85% BIT by weight based on the weight of the paste) was mixed with basic solution and with water. The amount of basic solution was chosen to have equivalent ratio of 1:1 with BIT, and the amount of water was chosen to make Mixture 1 have the desired concentration of BIT. In some cases, the process of mixing the ingredients of Mixture 1 is exothermic; in such cases, the peak temperature reached by the mixture was recorded as the "exotherm peak." From that mixture, a sample of 100 g (herein the "test1 batch") was used. If some or all of the BIT was not dissolved, the test1 batch was heated (up to a maximum of 61°C), and if there was a temperature of 61 °C or below at which all the BIT was dissolved, that temperature was recorded as the "dissolution temperature." If BIT is all dissolved at ambient temperature, the dissolution temperature is herein taken to be 23°C. Then, the inherent precipitation temperature was observed by cooling the test 1 batch to 0°C or to a point at which BIT precipitated, whichever temperature is higher. Then, the test 1 batch was heated to a temperature (herein "T2") at or above the dissolution temperature; while the solution was at temperature T2, thickener was added and acid solution was added.

In some of the Examples below, Protocol 1 was performed repeatedly, using various basic solutions and various concentrations of BIT. The purpose of these repeated performances of Protocol 1 was to answer the following question: "While using a specific basic solution and using temperatures between approximately 23°C and approximately 60°C, when making aqueous mixture (I), what range of BIT concentration is possible?"

"Protocol 2" means the following. Batch size was 1 kg, in a 3 liter beaker made of polypropylene. Agitation was performed with a Viscojet™ impeller of 120 mm diameter, rotation rate approximately 600 rpm (tangential velocity of the impeller tip was 3.77 m/sec). Aqueous mixture (I) was formed (BIT dissolved in water that also contained a base), using molar equivalent ratio of base to BIT of 0.94 to 1.02. Thickener was Xanthan gum (herein "Xanthan"). The glycol, if used, was triethylene glycol ("TEG"), which has the formula HO-(CH₂-O)₃-H. When glycol was used, it was mixed with Xanthan gum, and then the mixture of glycol and Xanthan gum added to the solution of BIT salt. The acid solution used was HCl, either concentrated ("conc. HCl," 34% HCl by weight, based on the weight of the acid solution) or diluted ("dil. HCl," 1:1 of water to concentrated acid, by weight). Then thickener and sometimes glycol were added; then acid was added to form the aqueous dispersion. The pH and viscosity of the aqueous dispersion were measured. Also, the stability of the aqueous dispersion was tested using a Heraeus Megafuge™ 16 centrifuge, for 15 minutes at 1650 rcf. The amount of sediment is reported as the volume of sediment in the centrifuge tube, as a percentage of the total volume of material in the centrifuge tube. Particle size distribution was measured with the Cilas device described herein above.

The purpose of Protocol 2 was to follow the method of the present invention and characterize the resulting aqueous dispersion.

In the tables below, if an amount for an ingredient is left blank, that ingredient was not used. If a result for a measurement is left blank, the measurement was not performed. The notation "nd" means not determined.

### Example 1: Solubility of BIT in NaOH solution

Protocol 1 was performed. The base solution was NaOH (50% by weight NaOH, based on the weight of the NaOH solution).

| Batch No. | 37a | 37c | 37d | 37e | 37g | 37h | 37i | 37k |
|---|---|---|---|---|---|---|---|---|
| BIT %⁽¹⁾ | 5 | 10 | 12.5 | 15 | 20 | 25 | 30 | 40 |
| Exotherm Peak (°C) | nd | nd | 27.9 | nd | 36.5 | 35.8 | 35.7 | nd |
| pH of Mixture 1 | 12.2 | 9.2 | 9.8 | 10.1 | 9.02 | 9.02 | 9.01 | ne |
| Dissolution Temp. (°C) | 23 | 23 | 27.9 | < 38 | 35.7 | 46 | 46 | 61 |
| Inherent Precipitation Temp. (°C) | -4.2 | 14.9 | 23.2 | 28.0 | 35.3 | 39.9 | 39.9 | 56.3 |

| | | | | | | | | |
|---|---|---|---|---|---|---|---|---|
| Note (1): % BIT by weight, based on the weight of the composition | | | | | | | | |

As the batches of Example 1 show, using NaOH, the BIT concentration range is from 5% to 40% by weight of BIT based on the weight of aqueous mixture (I). It is contemplated that Example 1 supports the conclusion that the method of the present invention could be practiced using BIT concentrations of 5% to 40%, because, over that concentration range, it was possible to make aqueous mixture (I) at a temperature of approximately 60°C or lower, and in each batch the dissolution temperature was higher than the inherent precipitation temperature. It is further contemplated that lower concentrations of BIT (e.g., 1% to 5%) could also be used.

### Example 2: Solubility of BIT in KOH solution

Protocol 1 was performed. The base solution was KOH (50% by weight KOH, based on the weight of the KOH solution).

| Batch No. | 38a | 38c | 38d | 38e | 38g | 38h | 38i | 38k |
|---|---|---|---|---|---|---|---|---|
| BIT %⁽¹⁾ | 5 | 10 | 12.5 | 15 | 20 | 25 | 30 | 40 |
| Exotherm Peak (°C) | 22.6 | 25.6 | 25.9 | 26.9 | 29.5 | 33.4 | 38.2 | nd |
| pH of Mixture 1 | 8.9 | 12.5 | 12.3 | 12.0 | 12.7 | 13.0 | 12.6 | nd |
| Dissolution Temp. (°C) | 23 | 23 | 23 | 23 | 29.5 | 48 | 45.2 | 68 |
| Inherent Precipitation Temp. (°C) | <-3 | 5 | 11 | 15 | 26 | 30 | 40 | 59 |

| | | | | | | | | |
|---|---|---|---|---|---|---|---|---|
| Note (1): % BIT by weight, based on the weight of the composition | | | | | | | | |

As the batches of Example 2 show, using KOH, the BIT concentration range is from 5% to 40% by weight of BIT based on the weight of aqueous mixture (I). It is further contemplated that lower concentrations of BIT (e.g., 1% to 5%) could also be used.

### Example 3: Solubility of BIT in mono-ethanolamine solution

Protocol 1 was performed. The base solution was mono-ethanolamine (pure).

| Batch No. | 39a |
|---|---|
| BIT %⁽¹⁾ | 5 |
| Exotherm Peak (°C) | 24.8 |
| pH of Mixture 1 | 9.1 |
| Dissolution Temp. (°C) | 58.5 |
| Inherent Precipitation Temp. (°C) | 62.5 |

| | |
|---|---|
| Note (1): % BIT by weight, based on the weight of the composition | |

The batch in Example 3 shows that, using mono-ethanolamine, the BIT concentration range includes 5%. It is contemplated that, because the dissolution temperature of 5% BIT in mono-ethanolamine solution was above 60°C, higher concentrations of BIT would require dissolution temperatures well above 60°C.

### Example 4: Solubility of BIT in diaminoethane solution

Protocol 1 was performed. The base solution was diaminoethane (pure).

| Batch No. | 40a |
|---|---|
| BIT %⁽¹⁾ | 5 |
| Exotherm Peak (°C) | 22.6 |
| pH of Mixture 1 | 8.6 |
| Dissolution Temp. (°C) | 70 |
| Inherent Precipitation Temp. (°C) | 56.6 |

| | |
|---|---|
| Note (1): % BIT by weight, based on the weight of the composition | |

The batch in Example 4 shows that, using diaminoethane, the BIT concentration range includes 5%. It is contemplated that, because the dissolution temperature of 5% BIT in diaminoethane solution was close to 60°C, higher concentrations of BIT would require dissolution temperatures well above 60°C.

### Example 5: Solubility of BIT in ammonia solution.

Protocol 1 was performed. The base solution was ammonia (25% ammonia by weight in water, based on the weight of the base solution).

| Batch No. | 40a |
|---|---|
| BIT %⁽¹⁾ | 5 |
| Exotherm Peak (°C) | 22.9 |
| pH of Mixture 1 | 9.3 |
| Dissolution Temp. (°C) | 63 |
| Inherent Precipitation Temp. (°C) | 59.3 |

| | |
|---|---|
| Note (1): % BIT by weight, based on the weight of the composition | |

The batch in Example 4 shows that, using ammonia, the BIT concentration range includes 5%. It is contemplated that, because the dissolution temperature of 5% BIT in ammonia solution was above 60°C, higher concentrations of BIT would require dissolution temperatures well above 60°C.

### Example 6: Solubility of BIT in LiOH solution

Protocol 1 was performed. The base solution was LiOH (pure solid).

| Batch No. | 42a | 42c | 42e | 42g | 42h | 42i | 42k |
|---|---|---|---|---|---|---|---|
| BIT %⁽¹⁾ | 5 | 10 | 15 | 20 | 25 | 30 | 40 |
| Exotherm Peak (°C) | 23.7 | 26.0 | 28.9 | 31.8 | 35.9 | 38.5 | nd |
| pH of Mixture 1 | 11.6 | 11.4 | 11.3 | 11.5 | 11.4 | 11.4 | nd |
| Dissolution Temp. (°C) | 23 | 23 | 23 | nd | 36 | 47 | >90 |
| Inherent Precipitation Temp. (°C) | nd | -2.8 | 13. | 19.8 | >24.6 | 30.3 | nd |

| | | | | | | | |
|---|---|---|---|---|---|---|---|
| Note (1): % BIT by weight, based on the weight of the composition | | | | | | | |

As the batches of Example 6 show, using LiOH, the BIT concentration range is from 5% to 30% by weight of BIT based on the weight of aqueous mixture (I). It is further contemplated that lower concentrations of BIT (e.g., 1% to 5%) could also be used. It is contemplated that 40% BIT could be used if LiOH solution had been used instead of LiOH pure solid.

### Example 7: 5% BIT, NaOH, HCl, Xanthan before acid.

Protocol 2 was followed. The amount of BIT was 5% by weight, based on the weight of the batch. The basic solution was NaOH (50% by weight NaOH, based on the weight of the basic solution).

| Batch No. | 43b | 43c | 43d | 43e | 43f⁽⁸⁾ | 43g⁽⁸⁾ | 43h | 43i | 43k |
|---|---|---|---|---|---|---|---|---|---|
| Xanthan⁽²⁾ (%) | 0.9 | 0.9 | 0.9 | 0.9 | 0.9 | 0.9 | 0.9 | 0.9 | 0.9 |
| TEG⁽²⁾(%) | | | | | | | 4 | 4 | 4⁽⁷⁾ |
| dil. HCl (g) | 78 | | | 78 | | 78 | 78.9 | | |
| conc. HCl (g) | | 39 | 39 | | 39 | | | 40.6 | 40.2 |
| HCl rate ⁽³⁾ | 1.42 | 31.17 | 12.5 | 6.4 | 43.6 | 1.4 | 1.3 | 14.2 | 13.6 |
| pH | 6.4 | 6.5 | 5.7 | 4.9 | 4.8 | 4.9 | 7.3 | 6.7 | 6.7 |
| viscosity (KU) | 68 | 68 | 69 | 68 | 70 | 68 | 68 | 70 | 68 |
| Sediment⁽⁴⁾ | 0 | 0 | 0 | 0 | 0 | 0 | 0 | 0 | 12 |
| PS mean⁽⁵⁾ | 9.8 | 9.0 | 9.1 | 11.1 | 13.0 | 12.4 | 12.6 | 12.4 | 25.8 |
| D99⁽⁶⁾ | 30 | 23 | 23 | 30 | 30 | 36 | 36 | 30 | 71 |

| | | | | | | | | | |
|---|---|---|---|---|---|---|---|---|---|
| Note (2): weight % based on the weight of the batch Note (3): acid addition rate in mole of acid per minute per kg of BIT Note (4): after centrifugation, weight % based on total BIT in centrifuge tube Note (5): Particle Size, weight-average diameter, micrometers. Note (6): Particle Size in micrometers, below which are 99% of all the solid particles of BIT. Note (7): also contained 0.5% Imbentin C/91/025 (dispersant from Dr. Kolb Company), by weight based on the weight of the batch Note (8): Stirring at 185 rpm (all others at 600 rpm) Note (13): see Example 17 for definitions of "HCl2" and Batch 48e | | | | | | | | | |

All the dispersions made in Example 7 are acceptable. Batch numbers 43f and 43g show that slower agitation yielded somewhat larger particles. Batch number 43k showed that addition of Imbentin C/91/025 dispersant led to larger particles (it is contemplated that other dispersants may have different effects on the process). In these batches, thickener was added before acid, and in these batches, the speed of addition of acid does not have a large effect.

### Example 8 (Comparative): 5% BIT, NaOH, HCl, acid before Xanthan

Protocol 2 was used, except that acid was added before the Xanthan.

| Batch No. | 44b | 44c | 44d | 44e | 44f |
|---|---|---|---|---|---|
| Xanthan⁽²⁾(%) | 0.9 | 0.9 | 0.9 | 0.9 | 0.9 |
| TEG⁽²⁾ (%) | 0 | 0 | 0 | 0 | 0 |
| dil. HCl (g) | 78 | | | 78 | 78 |
| conc. HCl (g) | | 39 | 39 | | |
| HCl rate⁽³⁾ | 1.4 | 31.2 | 14.15 | 1.5 | 6.4 |
| pH | 4.5 | nd | 6.3 | 4.1 | 4.7 |
| viscosity (KU) | 71 | 73 | 73 | 74 | 75 |
| Sediment⁽⁴⁾ | 15 | 0 | 0 | 15 | 0 |
| PS mean⁽⁵⁾ | 29.1 | 14.4 | 16.0 | 29.9 | 15.3 |
| D99⁽⁶⁾ | 90 | 56 | 71 | 90 | 45 |

| | | | | | |
|---|---|---|---|---|---|
| Notes (2) - (6): same as in Example 7 | | | | | |

The batches with acid addition rate of less than 2 asu yielded dispersions with undesirable sediment and with D99 higher than desired. The other batches had no sediment, as desired, and had somewhat smaller particle size. That is, when acid was added prior to thickener, it was preferable to add the acid relatively quickly. The sample in which acid addition rate was 6.4 asu, the acid was also diluted prior to addition to aqueous mixture (I); it is contemplated that the combination of dilute acid and quick addition leads to low sediment and small particle size.

### Example 9: 20% BIT, NaOH, HCl, Xanthan before acid.

Protocol 2 was followed, except that the batch size was 1 kg. The amount of BIT was 20% by weight, based on the weight of the batch. The basic solution was NaOH (50% by weight NaOH, based on the weight of the basic solution).

| Batch No. | 45a | 45b | 45c |
|---|---|---|---|
| Xanthan⁽²⁾ (%) | 0.9 | 0.9 | 0.9 |
| TEG⁽²⁾ (%) | 0 | 0 | 0 |
| dil. HCl (g) | 297 | 272 | |
| conc. HCl (g) | | | 136 |
| HCl rate ⁽³⁾ | 0.35 | 0.35 | 10.6 |
| pH | 7.1 | 8.5 | 8.54 |
| viscosity (KU) | 90 | 93 | 100 |
| Sediment⁽⁴⁾ | 0 | 0 | 0 |
| PS mean⁽⁵⁾ | 17.3 | 15.2 | 14.7 |
| D99^{(b)} | 56 | 45 | 45 |

| | | | |
|---|---|---|---|
| Note (2) - (6): same as in Example 7 | | | |

The batches produced in Example 9 were acceptable, though the particles were larger than desired. The method of the present invention operates properly at BIT concentration of 20%.

### Example 10 (Comparative): 20% BIT, NaOH, HCl, acid before Xanthan

Protocol 2 was followed, except that the batch size was 1 kg, and the acid was added to form the aqueous dispersion before the thickener was added. The amount of BIT was 20% by weight, based on the weight of the batch. The basic solution was NaOH (50% by weight NaOH, based on the weight of the basic solution).

| Batch No. | 46a | 46b | 46c |
|---|---|---|---|
| Xanthan⁽²⁾(%) | 0.9 | 0.9 | 0.9 |
| TEG⁽²⁾ (%) | 0 | 0 | 0 |
| dil. HCl (g) | 297 | | |
| conc. HCl (g) | | 142 | 142 |
| HCl rate⁽³⁾ | 0.38 | 3.26 | 11.4 |
| pH | 8.5 | 8.5 | 8.6 |
| viscosity (KU) | 92 | 99 | 101 |
| Sediment⁽⁴⁾ | 35 | 0 | 0 |
| PS mean⁽⁵⁾ | 49.3 | 33.4 | 28.0 |
| D99⁽⁶⁾ | 140 | 90 | 90 |

| | | | |
|---|---|---|---|
| Note (2) - (6): same as in Example 7 | | | |

Batches 46b and 46c were just acceptable dispersions, though the particles were larger than desired. Batch 46a (which used very slow rate of acid addition) had too large D99 and too much sediment to be acceptable. It is contemplated that the relatively high viscosity contributed to the stability (i.e., lack of sediment) in Batches 46b and 46c.

### Example 11: 5% BIT, NaOH, HCl, varied amount of Xanthan.

Protocol 2 was followed. The amount of BIT was 5% by weight, based on the weight of the batch. The basic solution was NaOH (50% by weight NaOH, based on the weight of the basic solution).

| Batch No. | 47a | 47b | 48a | 48b |
|---|---|---|---|---|
| Xanthan⁽²⁾ (%) | 0.3 | 0.3 | 0.6 | 0.6 |
| TEG⁽²⁾ (%) | | | | |
| dil. HCl (g) | 78 | | 78 | |
| conc. HCl (g) | | 39 | | 39 |
| HCl rate ⁽³⁾ | 1.4 | 13.2 | 1.4 | 12.1 |
| pH | 7.2 | 7.4 | 7.0 | 7.2 |
| viscosity (KU) | 53 | 52 | 58 | 58 |
| Sediment⁽⁴⁾⁽⁹⁾ | 13 | 13 | 0 | 0 |
| PS mean⁽⁵⁾ | 12.9 | 11.2 | 10.6 | 9.7 |
| D99⁽⁶⁾ | 36 | 30 | 30 | 30 |

| | | | | |
|---|---|---|---|---|
| Note (2) - (6): same as in Example 7 | | | | |

All the batches in Example 11 had acceptable particle size. The samples with higher amount of Xanthan gum had no sediment, which is more desirable.

### Example 12: 5% BIT, NaOH, different acids

Protocol 2 was followed. The amount of BIT was 5% by weight, based on the weight of the batch. The basic solution was NaOH (50% by weight NaOH, based on the weight of the basic solution). The acid was either acetic acid or formic acid.

| Batch No. | 49a | 49b |
|---|---|---|
| Xanthan⁽²⁾ (%) | 0.9 | 0.9 |
| TEG⁽²⁾ (%) | 0 | 0 |
| acetic acid (g) | 19.1 | |
| formic acid (g) | | 17.2 |
| acid rate⁽³⁾ | 0.92 | 1.07 |
| pH | 7.4 | 7.3 |
| viscosity (KU) | 70 | 69 |
| Sediment⁽⁴⁾⁽⁹⁾ | 0 | 0 |
| PS mean⁽⁵⁾ | 10.2 | 11.2 |
| D99⁽⁶⁾ | 30 | 36 |

| | | |
|---|---|---|
| Note (2) - (6): same as in Example 7 Note (9): Centrifuge method: 3x 5 min at 1650 rcf | | |

Both samples in Example 12 were acceptable, which shows that the method of the present invention may be practiced using organic acids.

### Example 13: 10% BIT, NaOH, HCl, Xanthan before acid

Protocol 2 was followed. The amount of BIT was 10% by weight, based on the weight of the batch. The basic solution was NaOH (50% by weight NaOH, based on the weight of the basic solution).

| Batch No. | 50a | 50b |
|---|---|---|
| Xanthan⁽²⁾ (%) | 0.9 | 0.9 |
| TEG⁽²⁾ (%) | | |
| dil. HCl (g) | 156 | |
| conc. HCl (g) | | 78 |
| HCl rate⁽³⁾ | 1.4 | 13.9 |
| pH | 7.0 | 6.7 |
| viscosity (KU) | 75 | 76 |
| Sediment⁽⁴⁾⁽⁹⁾ | 0 | 0 |
| PS mean⁽⁵⁾ | 12.9 | 13.4 |
| D99⁽⁶⁾ | 45 | 36 |

| | | |
|---|---|---|
| Note (2) - (6): same as in Example 7 | | |

Both samples in Example 13 were acceptable, which shows that the method of the present invention may be practiced using 10% BIT.

### Example 14: variable % BIT, KOH, HCl, Xanthan before acid

Protocol 2 was followed. The amount of BIT was 5% or 10% by weight, based on the weight of the batch. The basic solution was KOH (50% by weight KOH, based on the weight of the basic solution).

| Batch No. | 51a | 51b | 51c | 51d |
|---|---|---|---|---|
| BIT (%) | 5 | 5 | 10 | 10 |
| Xanthan⁽²⁾ (%) | 0.9 | 0.9 | 0.9 | 0.9 |
| TEG⁽²⁾ (%) | | | | |
| dil. HCl (g) | 84.7 | | 156.8 | |
| conc. HCl (g) | | 39.5 | | 78.6 |
| HCl rate⁽³⁾ | 1.3 | 14.7 | 0.66 | 6.5 |
| pH | 7.0 | 6.9 | 7.2 | 7.1 |
| viscosity (KU) | 68 | 68 | 74 | 74 |
| Sediment⁽⁴⁾ | 0 | 0 | 0 | 0 |
| PS mean⁽⁵⁾ | 10.2 | 10.2 | 9.8 | 10.2 |
| *D99⁽⁶⁾ | 30 | 30 | 36 | 20 |

| | | | | |
|---|---|---|---|---|
| Note (2) - (6): same as in Example 7 | | | | |

Both samples in Example 14 were acceptable, which shows that the method of the present invention may be practiced using KOH. The two samples with slowest acid addition rate also used dilute acid, and the resulting dispersions were acceptable.

### Example 15: 5% BIT, LiOH, HCl, Xanthan before precipitation

Protocol 2 was followed. The amount of BIT was 5% by weight, based on the weight of the batch. The basic solution was LiOH (added as a pure solid).

| Batch No. | 52a | 52b |
|---|---|---|
| Xanthan⁽²⁾ (%) | 0.9 | 0.9 |
| TEG⁽²⁾ (%) | | |
| dil. HCl (g) | 78 | |
| conc. HCl (g) | | 39.5 |
| HCl rate⁽³⁾ | 1.3 | 11.9 |
| pH | 6.5 | 6.6 |
| viscosity (KU) | 67 | 70 |
| Sediment⁽⁴⁾⁽⁹⁾ | 0 | 0 |
| PS mean⁽⁵⁾ | 10.8 | 11.2 |
| D99⁽⁶⁾ | 30 | 30 |

| | | |
|---|---|---|
| Note (2) - (6): same as in Example 7 | | |

Both samples in Example 15 were acceptable, which shows that the method of the present invention may be practiced using LiOH.

### Example 16: 5% BIT, NaOH, HCl, non-Xanthan thickener before acid

Protocol 2 was followed. The amount of BIT was 5% by weight, based on the weight of the batch. The basic solution was NaOH (50% by weight NaOH, based on the weight of the basic solution). The Xanthan was replaced by Cellosize ™ QP 52000H thickener (herein "HEC1"), which is a hydroxyethyl cellulose, a high molecular weight cellulosic polymer.

| Batch No. | 53a | 53b | 53c | 53d | 53e⁽¹⁰⁾ | 53f⁽¹¹⁾ |
|---|---|---|---|---|---|---|
| HEC1⁽²⁾ (%) | 1 | 1 | 0.6 | 0.8 | 0.8 | 0.8 |
| dil. HCl (g) | 78.5 | | | 78.4 | 79.3 | 79.4 |
| conc. HCl (g) | | 39.5 | 39.4 | | | |
| HCl rate⁽³⁾ | 1.2 | 14.7 | 13.4 | 1.3 | 1.3 | 1.3 |
| pH | 6.5 | 6.1 | 4.8 | 7.4 | 7.1 | 7.2 |
| viscosity (KU) | 89 | 90 | 56 | 68 | 70 | 78 |
| Sediment⁽⁴⁾⁽⁹⁾ | 0 | 0 | 10 | 0 | 10 | 12 |
| PS mean⁽⁵⁾ | 11.8 | 19.1 | 18.1 | 11.6 | 16.3 | 29.3 |
| D99⁽⁶⁾ | 30 | 56 | 45 | 30 | 71 | 38.6 |

| | | | | | | |
|---|---|---|---|---|---|---|
| Note (2) - (6): same as in Example 7 Note (10): Also contained 4% triethylene glycol, by weight based on the weight of the aqueous dispersion. Note (11): also contains 0.25% by weight based on the weight of the batch of Imbentin C/91/025. | | | | | | |

The batches in Example 16 were all acceptable, though the sediment and particle size were not as desirable in the sample with Imbentin C/91/025.

### Example 17 (Comparative): 5% BIT, NaOH, HCl, Xanthan, alternative protocol

Batch size was 1 kg. Water was municipal tap water. NaOH 50% solution was 50% NaOH by weight, based on the weight of the NaOH solution. BIT paste and conc. HCl were the same as defined above in Protocols 1 and 2.

The following mixtures were made:

| | |
|---|---|
| BIT20 | 65 pbw water, 24 pbw BIT paste, and 11 pbw NaOH 50% solution |
| BIT10 | 82.6 pbw water, 12.0 pbw BIT paste, and 5.4 pbw NaOH 50% solution |
| HCl2 | 5.59 pbw conc. HCl and 94.4 pbw water |
| HCl3 | from 8.6 pbw conc. HCl and 91.4 pbw water |
| X/TEG | 26.5 pbw Xanthan gum and 73.5 pbw triethylene glycol |

Both BIT20 and BIT10 were made at 50°C and held at that temperature until use.

Batch 58a was made as follows. Premix58a was 3.4 pbw of X/TEG plus 71.6 pbw of HCl2. Then, 25 pbw of BIT20 was poured into a vessel containing 75 pbw of Premix58a, while the vessel was stirred as in Protocol 2 at 600 rpm. The pouring was performed by hand at a constant rate over 60 seconds.

Batch 58b was made as follows: Premix 58b was 3.40 pbw of X/TEG and 46.65 pbw of HCl3. Then, 49.95 pbw of BIT10 was poured into a vessel containing 50.05 pbw of Premix 58b, while the vessel was stirred as in Protocol 2 at 600 rpm. The pouring was performed by hand at a constant rate over 60 seconds.

Batch 58d was made as follows: 25.0 pbw of BIT20 was poured into a vessel containing 71.6 pbw of HCl2, while the vessel was stirred as in Protocol 2 at 600 rpm. The pouring was performed by hand at a constant rate over 60 seconds. Then 3.4 pbw of X/TEG was added to the vessel, as stirring continued.

Batch 58e was made as follows: Premix58e was 3.4 pbw of X/TEG plus 71.6 pbw of HCl2. Then, premix 58e was poured into a vessel containing 25 pbw of BIT20, while the vessel was stirred as in Protocol 2 at 600 rpm. The pouring was performed by hand at a constant rate over 60 seconds.

The resulting characteristics of each batch were as follows.

| Batch No. | 58a | 58b | 58d | 58e |
|---|---|---|---|---|
| pH | 4.16 | 7.13 | 5.02 | 7.25 |
| viscosity (KU) | 66 | 68 | 71 | 75 |
| Sediment⁽⁴⁾ | 0 | 10%⁽²⁾ | 0 | 10%⁽¹²⁾ |
| PS mean⁽⁵⁾ | 25.8 | 26.3 | 11.3 | 15.3 |
| D99⁽⁶⁾ | 90 | 90 | 36 | 56 |

| | | | | |
|---|---|---|---|---|
| Notes (4) - (6) are the same as in Example 7. Note (12): sediment was not as firmly packed as sediment that is observed in other batches that show sediment. Stability is considered to be better than is usually associated with a result of 10%. | | | | |

The batches in Example 17 all were acceptable, though the particles were larger than desired. Pouring concentrated solution of dissolved salt of BIT into diluted acid solution (as in batches 58a, 58b, and 58d) enables an acceptable dispersion in which the solid particles of BIT are acceptably small; particle size is even smaller when (as in batch 58d), thickener was not present during the precipitation step. The relatively low level of sediment (especially in Batches 58a and 58d) is desirable.

### Example 18: High-I Examples

Various examples of aqueous mixture (I) were formed. The ingredients used were as follows: 640 g city water; 48 g BIT paste (concentration 84.8%); 2.24 g NaOH (concentration 50%); 7.20 g Xanthan gum. If these aqueous mixtures were to be used to make the aqueous dispersion of the present invention, acid would be added to the aqueous mixtures. Example 18-A could either be considered to be a low-I embodiment of aqueous mixture (I) followed by addition of Xanthan gum or considered to be a high-I embodiment of aqueous mixture (I) in which Xanthan gum was added after BIT and base. Examples 18-B, 18-C, 18-CR, and 18-D are high-I embodiments.

| | **Example 18-A** | **Example 18-B** | **Example 18-C** | **Example 18-CR** | **Example 18-D** |
|---|---|---|---|---|---|
| Order of addition | water, BIT, NaOH, Xanthan | water, BIT, Xanthan, NaOH | water, Xanthan, BIT, NaOH | water, Xanthan, BIT, NaOH | water, Xanthan, NaOH, BIT |
| stirrer speed | 500 rpm | 500 rpm | 500 rpm | 750 rpm | 500 rpm |
| time to complete dissolution | 110 min | 60 min | 80 min | 50 min | 45 to 90 min |
| pH | 9.52 | 9.18 | 11.97 | 11.95 | 9.85 |
| appearance | a few lumps | homogeneous | homogeneous | homogeneous | homogeneous |
| sediment⁽¹³⁾ | 0.3 ml | 0.2 ml | 0 ml | expect 0⁽¹⁵⁾ | 0.3 ml |
| residue⁽¹⁴⁾ | moderate | high | almost none | none | small |

| | | | | | |
|---|---|---|---|---|---|
| Note 13: volume of sediment after centrifugation for 8 min. at 45000 rcb. Note 14: amount observed on stir shaft after dissolution Note 15: It is expected that Example 18-CR would exhibit 0 ml of sediment after centrifugation, because Example 18-CR is the same as Example 18-C except for higher stirrer speed. | | | | | |

All five examples are useable for making the aqueous dispersion of the present invention. Examples 18-B, 18-C, 18-CR, and 18-D dissolved more quickly and had homogeneous appearance. Example 18-C and Example 18-CR had acceptable dissolution time, were homogeneous, and had the best level of sediment and residue. Example 18-CR also had desirably short dissolution time.

### Example 19: pH adjustment

Further NaOH solution was added to Examples 18-A, 18-B, and 18-D. The final pH is shown below, along with the sediment (as assessed in Example 18).

| | **Example 19-A** | **Example 19-B** | **Example 19-D** |
|---|---|---|---|
| starting material: | Example 18-A | Example 18-B | Example 18-D |
| final pH | 12.09 | 11.08 | 11.97 |
| sediment | 0.1 to 0.2 ml | 0 | less than 0.1 ml |

It is contemplated that raising the pH of Examples 18-A, 18-B, and 18-D decreased the sediment. It is further contemplated that if sufficient base were added to the mixture during the initial formation of aqueous mixture (I) so that the pH of the resulting aqueous mixture (I) were 11.0 or higher, that formulations analogous to Examples 18-A, 18-B, and 18-D would show reduced or eliminated sediment and/or residue. Additionally, it is also contemplated that if sufficient base were added to the mixture during the initial formation of aqueous mixture (I) so that the pH of the resulting aqueous mixture (I) were 11.0 or higher, that formulations analogous to Examples 18-A, 18-B, and 18-D would show reduced dissolution time.

### Example 20: Formation of Aqueous Dispersion

Example 18-CR was used to form an aqueous dispersion. HCl at concentration 30 to 34% (by weight based on the weight of the HCl solution) was added over 8 seconds at a rate of 57.4 mmol of acid per minute per kilogram of BIT. Final pH was 2.10. Viscosity was 73 KU. Particle size mean (assessed as in Example 7) was 11.34 micrometers. This aqueous dispersion was considered acceptable.

## Claims

1. A method of making an aqueous dispersion of BIT particles having a D99 particle size distribution, measured using laser light scattering, of 75 microns or smaller, wherein said dispersion comprises
(A) one or more thickener and
(B) solid particles of 1,2-benzisothiazolin-3-one,
wherein said method comprises the steps of
(a) forming an aqueous mixture (I) comprising water and a dissolved salt of 1,2-benzisothiazolin-3-one, wherein the pH of said aqueous mixture (I) is 8.5 or higher, and
(b) subsequently forming said aqueous dispersion by mixing together, in any order, components, wherein said components comprise an acid and said aqueous mixture (I), wherein the pH of said dispersion is 1.5 to 7.5,
provided that either:
(i) aqueous mixture (I) contains said one or more thickener,
or
(ii) step (b) comprises:
(b1) making a mixture comprising said one or more thickener and said aqueous mixture (I), and
(b2) making a mixture comprising said acid and said aqueous mixture (I),
wherein the amount of said acid is sufficient to bring said mixture comprising said acid and said aqueous mixture (I) to a pH of 1.5 to 7.5,
wherein said step (b1) is performed prior to said step (b2).

2. The method of claim 1, wherein said step (a) comprises the steps of
(a1) forming a mixture (III) of water, 1,2-benzisothiazolin-3-one, and one or more thickener, wherein the pH of said mixture (III) is 7.5 or lower, and
(a2) then raising the pH of said mixture (III) to 8.5 or higher.

3. The method of claim 1, wherein the amount of said 1,2-benzisothiazolin-3-one is 1% to 40% by weight based on the weight of said aqueous dispersion.

## Patentansprüche

1. Ein Verfahren zum Herstellen einer wässrigen Dispersion von BIT-Partikeln mit einer D99-Partikelgrößenverteilung, gemessen unter Verwendung von Laserlichtstreuung, von 75 Mikrometer oder weniger, wobei die Dispersion Folgendes beinhaltet:
(A) ein oder mehrere Verdickungsmittel und
(B) feste 1,2-Benzisothiazolin-3-on-Partikel,
wobei das Verfahren die folgenden Schritte beinhaltet:
(a) Bilden einer wässrigen Mischung (I), die Wasser und ein gelöstes Salz von 1,2-Benzisothiazolin-3-on beinhaltet, wobei der pH-Wert der wässrigen Mischung (I) 8,5 oder mehr beträgt, und
(b) nachfolgendes Bilden der wässrigen Dispersion durch das Zusammenmischen, in jeder beliebigen Reihenfolge, von Komponenten, wobei die Komponenten eine Säure und die wässrige Mischung (I) beinhalten, wobei der pH-Wert der Dispersion 1,5 bis 7,5 beträgt,
vorausgesetzt, dass entweder:
(i) die wässrige Mischung (I) das eine oder die mehreren Verdickungsmittel enthält,
oder
(ii) Schritt (b) Folgendes beinhaltet:
(b1) Herstellen einer Mischung, die das eine oder die mehreren Verdickungsmittel und die wässrige Mischung (I) beinhaltet, und
(b2) Herstellen einer Mischung, die die Säure und die wässrige Mischung (I) beinhaltet,
wobei die Menge der Säure ausreicht, um die Mischung, die die Säure und die wässrige Mischung (I) beinhaltet, auf einen pH-Wert von 1,5 bis 7,5 zu bringen,
wobei der Schritt (b1) vor Schritt (b2) durchgeführt wird.

2. Verfahren gemäß Anspruch 1, wobei der Schritt (a) die folgenden Schritte beinhaltet:
(a1) Bilden einer Mischung (III) aus Wasser, 1,2-Benzisothiazolin-3-on und einem oder mehreren Verdickungsmitteln, wobei der pH-Wert der Mischung (III) 7,5 oder niedriger ist, und
(a2) dann Erhöhen des pH-Werts der Mischung (III) auf 8,5 oder mehr.

3. Verfahren gemäß Anspruch 1, wobei die Menge des 1,2-Benzisothiazolin-3-on 1 Gew.-% bis 40 Gew.-%, bezogen auf das Gewicht der wässrigen Dispersion, beträgt.

## Revendications

1. Une méthode de réalisation d'une dispersion aqueuse de particules de BIT ayant une distribution de tailles de particules D99, mesurée en utilisant une diffusion de lumière laser, de 75 microns ou moins, dans laquelle ladite dispersion comprend
(A) un ou plusieurs épaississants et
(B) des particules solides de 1,2-benzisothiazolin-3-one,
ladite méthode comprenant les étapes consistant à
(a) former un mélange aqueux (I) comprenant de l'eau et un sel dissous de 1,2-benzisothiazolin-3-one, le pH dudit mélange aqueux (I) étant de 8,5 ou plus, et
(b) former, subséquemment, ladite dispersion aqueuse en mélangeant ensemble, dans un ordre quelconque, des constituants, lesdits constituants comprenant un acide et ledit mélange aqueux (I), le pH de ladite dispersion allant de 1,5 à 7,5,
à condition que soit :
(i) le mélange aqueux (I) contient lesdits un ou plusieurs épaississants,
soit
(ii) l'étape (b) comprend :
(b1) la réalisation d'un mélange comprenant lesdits un ou plusieurs épaississants et ledit mélange aqueux (I), et
(b2) la réalisation d'un mélange comprenant ledit acide et ledit mélange aqueux (I),
la quantité dudit acide étant suffisante pour amener ledit mélange comprenant ledit acide et ledit mélange aqueux (I) à un pH de 1,5 à 7,5,
ladite étape (b1) étant effectuée préalablement à ladite étape (b2).

2. La méthode de la revendication 1, dans laquelle ladite étape (a) comprend les étapes consistant à
(a1) former un mélange (III) d'eau, de 1,2-benzisothiazolin-3-one, et d'un ou plusieurs épaississants, le pH dudit mélange (III) étant de 7,5 ou moins, et
(a2) élever ensuite le pH dudit mélange (III) à 8,5 ou plus.

3. La méthode de la revendication 1, dans laquelle la quantité de ladite 1,2-benzisothiazolin-3-one représente de 1 % à 40 % en poids rapporté au poids de ladite dispersion aqueuse.
